# EUROPÄISCHE PATENTSCHRIFT

(11) **EP 1 319 411 B1**
(45) Veröffentlichungstag und Bekanntmachung des Hinweises auf die Patenterteilung: **16.09.2009**
(21) Anmeldenummer: 02026778.7
(22) Anmeldetag: 02.12.2002
(51) Int. Cl.: A61L 2/28, A61L 2/18, G05D 7/06, G05D 23/19, D06F 39/04, D06F 39/00, A47L 15/42

(54) **Reinigungs- und Desinfektionsmaschine**
Cleaning and disinfecting machine
Machine à nettoyer et désinfecter

(30) Priorität: 12.12.2001 DE 10161106
(43) Veröffentlichungstag der Anmeldung: 18.06.2003
(60) Teilanmeldung: 08016545.9
(73) Patentinhaber: BHT Hygienetechnik GmbH, 86316 Friedberg/Derching (DE)
(72) Erfinder: Annecke, Karl Heinz, Dr., 64625 Bensheim (DE)
(74) Vertreter: von Bülow, Tam

(56) Entgegenhaltungen:
- EP-A- 1 000 629
- EP-A- 1 130 381
- DE-A- 3 705 596
- US-A- 4 843 867
- US-A1- 2001 033 805

## Beschreibung

Die Erfindung bezieht sich auf eine Reinigungs- und Desinfektionsmaschine gemäß dem Oberbegriff des Patentanspruches 1. Solche Maschinen sind allgemein bekannt und werden von der Anmelderin seit Jahren vertrieben.

Die EP 1 130 381 A1 beschreibt eine Analysevorrichtung für die Messung des Reinheitsgrades von Wasser. Die Vorrichtung hat eine Meßkammer mit einem Einlaß und einem Auslaß. In der Kammer sind mehrere optische Sensoren angeordnet, die den Transmissionsgrad des Wassers messen. Zusätzlich kann dem Einlaß ein Durchflußmesser vorgeschaltet sein. Die drei Sensoren für die Messung des Transmissionsgrades arbeiten nach demselben physikalischen Meßprinzip.

Die US 2001/0033805 A1 beschreibt ein Reinigungsverfahren für medizinische Geräte mit einer Reinigungskammer, die einen Einlaß und einen Auslaß hat und daher von einer Reinigungsflüssigkeit durchströmt werden kann. Die Schrift schlägt eine Vielzahl möglicher Sensoren vor, mit denen Verunreinigungen, wie z.B. organische Verunreinigungen, anorganische Verunreinigungen etc., des die Vorrichtung durchfließenden Reinigungsmittels gemessen werden können, so daß in einigen Ausführungsbeispielen auch die Anwendung mehrerer unterschiedlicher Sensoren vorgeschlagen wird, mit denen Proteine und andere organische Substanzen sowie anorganische Substanzen detektiert werden können.

Im Zusammenhang mit der Durchsetzung des Medizinproduktegesetzes und den daraus entstehenden Ausführungsverordnungen und Normen für Reinigungs- und Desinfektionsmaschinen wird das Thema "Validierung des Reinigungs-/Desinfektionsverfahrens" bzw. Validierung der Reinigung und Desinfektion gefahrener Parameter zunehmend wichtiger. Validierung heißt, daß eine physikalische Größe (z.B. Volumen, Temperatur, Zeit oder ähnliches) auf der einen Seite durch eine Steuerung und dazu notwendige Sensoren so eingestellt wird, daß die an den Wasch- und Desinfektionsprozeß gestellten Anforderungen erfüllt werden. Andererseits muß aber durch einen davon unabhängigen Sensor bzw. ein davon unabhängiges Meßverfahren zwecks Validierung bzw. dann auch nachfolgender Dokumentation festgestellt werden, ob die der Steuerung eingegebenen Sollwerte tatsächlich während des Wasch- und Desinfektionsprozesses eingehalten wurden. Dies heißt beispielsweise bei der Desinfektionstemperatur, daß die Steuerung, die die Heizung zum Erreichen der notwendigen Desinfektionstemperatur ein- und ausschaltet, auf der einen Seite einen Temperaturfühler besitzt und daß auf der anderen Seite ein ähnlich oder gleicher Temperaturfühler in der Kammer angebracht ist, der überprüft, ob die vorgegebene Temperatur tatsächlich erreicht wurde und der dieses beispielsweise in einem Protokollausdruck niederlegt.

Nachteil bei der Verwendung von in diesem Falle zwei gleichartigen Temperaturfühlern und eventuell auch gleichen Strecken in der Auswertung (z.B. Analog/Digitalwandler; Mikrocontroller usw.) ist, daß sich z.B. Serienfehler in den Temperaturfühlern oder auch Verarbeitungsfehler auf der weiteren Verarbeitungsstrecke dahingehend auswirken, daß die Steuerung auf der einen Seite die verkehrte Temperatur einstellt und der Validierungsfühler auf der anderen Seite aber nicht feststellt, daß die Solltemperatur nicht erreicht wurde, da er ebenfalls die Auswertung mit dem gleichen Fehler behaftet vornimmt.

Aufgabe der Erfindung ist es daher, die bekannte Reinigungs- und Desinfektionsmaschine dahingehend zu verbessern, daß eine höhere Sicherheit der Validierung erreicht wird.

Diese Aufgabe wird durch die im Patentanspruch angegebenen Merkmale gelöst.

Grundprinzip der Erfindung ist es, für die jeweils zu erfassende Messung der Menge einer Flüssigkeit zwei Meßfühler zu verwenden, die auf unterschiedlichen physikalischen Meßverfahren beruhen.

Beispielsweise wird für die Dosierung von Chemikalien heute eine geeignete Dosierpumpe, wie z.B. eine Schlauchpumpe, verwendet, die eine vorgegebene Zeit läuft und damit innerhalb gewisser Toleranzen eine vorgegebene Menge von Chemikalien der Waschkammer zuführt. Eine Meßgröße, die der Dosiermenge entspricht, ist somit die Laufzeit der Dosierpumpe. Die zweite hiervon vollkommen unabhängige Meßmethode liegt in einem Durchflußmesser mit hoher Meßgenauigkeit. Damit können sich die beiden Meßmethoden gegenseitig kontrollieren und, da beide unterschiedlichen physikalischen Meßverfahren unabhängig voneinander sind, Fehler des einen Meßverfahrens erkannt werden. Würde man beispielsweise lediglich zwei hintereinander geschaltete Durchflußmesser verwenden, die beide also auf dem gleichen physikalischen Meßprinzip basieren, so würden systematische Fertigungsfehler der Durchflußmesser oder Auswertefehler der Impulse von beiden Durchflußmessern ein falsches Signal liefern und der Prozeß wäre nicht sicher validierbar.

Wie bekannt, spielt in der Reinigungstechnik nicht nur die absolute Menge des Reinigungs- bzw. Desinfektionsmittels eine Rolle, sondern auch die prozentuale Beimischung zum zugelassenen Wasser, so daß eine weitere physikalische Größe, die möglichst exakt ermittelt werden sollte, die zugeführte Wassermenge ist. Auch hier gibt es wieder verschiedene Möglichkeiten, die zugeführte Wassermenge durch zwei unterschiedliche physikalische Parameter zu erfassen.

Im folgenden wird die Erfindung anhand eines Ausführungsbeispieles im Zusammenhang mit der Zeichnung ausführlicher erläutert.

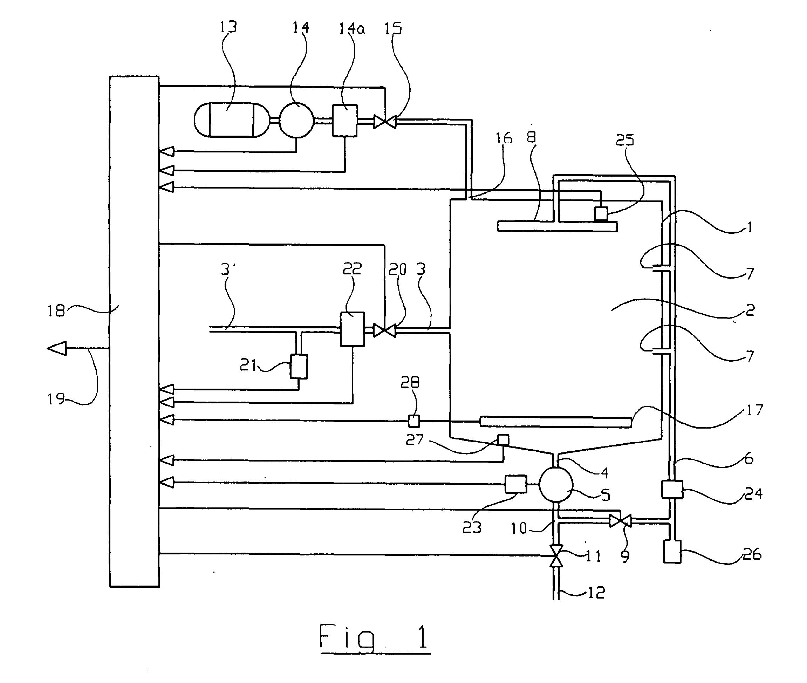

Die einzige Figur 1 zeigt ein Prinzipschaltbild einer Reinigungs- und Desinfektionsmaschine nach der Erfindung.

Die Reinigungs- und Desinfektionsmaschine, im folgenden "Waschmaschine 1" genannt, hat einen Waschraum 2 mit einem Frischwassereinlaß 3, einem Auslaß 4, einem Umwälzkreislauf mit einer Umwälzpumpe 5, einer Umwälzleitung 6, die über ein oder mehrere Düsen 7 und/oder einen oder mehrere rotierende Wascharme 8 in den Waschraum 2 mündet, wobei in der Umwälzleitung ein elektrisch steuerbares Ventil 9 vorgesehen ist. An den Ausgang der Umwälzpumpe 5 ist ein Verzweigungsstück 10 angeschlossen, das einerseits mit der Umwälzleitung 6 und andererseits über ein Ventil 11 mit einem Abwasserkanal 12 verbunden ist.

Weiter ist ein Vorratsbehälter 13 für Reinigungs- und/oder Desinfektionsmittel vorgesehen, der über eine Dosierpumpe 14 und ein Ventil 15 mit einem Einlaß 16 in den Waschraum 2 der Maschine verbunden ist.

Im Inneren des Waschraumes ist eine Heizeinrichtung 17 angeordnet, die üblicherweise elektrisch betrieben wird.

Der gesamte Reinigungs- und Desinfektionsvorgang wird von einer Steuereinheit 18 gesteuert und überwacht, die an einem Ausgang 19 auch Protokolldaten über sämtliche Abläufe ausgeben kann, womit das Reinigungs- und Desinfektionsergebnis validiert wird.

Insoweit handelt es sich bisher um ein Beispiel einer herkömmlichen Waschmaschine.

Um ein einwandfreies, reproduzierbares und nachweisbares Reinigungs- und Desinfektionsergebnis erzielen zu können, müssen sämtliche Arbeitsabläufe überwacht und dokumentiert werden. Hierzu sind eine Vielzahl von Meßfühlern vorgesehen, mit denen diverse Parameter gemessen werden und von der Steuerung ausgewertet werden. Beispielsweise sind folgende Parameter zu überwachen:
- Frischwassermenge
- Menge des Reinigungs- und Desinfektionsmittels
- Menge des umgewälzten aktuellen Waschwassers
- Temperatur des Waschwassers
- Menge des Abwassers.

Alle bekannten Sensoren für diese Größen können fehlerhafte Meßergebnisse liefern. Verwendet man zur Überprüfung der Meßergebnisse jeweils einen weiteren gleichartigen Sensor, so können sich z.B. Serienfehler der Sensoren oder auch Verarbeitungsfehler dahingehend auswirken, daß die Steuerung auf der einen Seite einen verkehrten Wert einstellt und der zweite Sensor, der als Validierungsfühler dient, auf der anderen Seite aber nicht feststellt, daß der Sollwert nicht erreicht wurde, da dessen Auswertung mit dem gleichen Fehler behaftet ist. Der wesentliche Kerngedanke der Erfindung liegt darin, für die Regelung bzw. Steuerung des jeweiligen Parameters und für dessen Überprüfung bzw. Validierung jeweils Meßfühler zu verwenden, die auf einem unterschiedlichen physikalischen Meßprinzip basieren. Dies wird nun im folgenden anhand verschiedener Parameter erläutert.

### Frischwasserzufuhr (nicht Gegenstand der Erfindung)

Die Frischwasserzufuhr von dem Frischwassereinlaß 3' wird über ein Ventil 20 gesteuert. Anhand der Öffnungszeit des Ventiles, dem von einem Drucksensor 21 gemessenen Wasserdruck und bekannten Parametern, wie Leitungsquerschnitte, Strömungswiderstand etc. läßt sich die zugeführte Frischwassermenge ermitteln. Als ein auf anderem physikalischen Meßprinzip basierender Sensor ist ein Durchflußmesser 22 vorgesehen, der in der Zuleitung zum Frischwassereinlaß 3 angeordnet ist und die Wassermenge mißt. Die beiden aufgrund unterschiedlicher physikalischer Meßmethoden ermittelten Meßwerte können verglichen werden, so daß sich beide Sensoren wechselseitig überwachen.

### Dosierung von Reinigungs- und Desinfektionsmitteln

Als erster Sensor dient hier die Dosierpumpe 14, deren Umdrehungszahl proportional der geförderten Menge ist. Wird für den Antrieb beispielsweise ein Motor mit Umdrehungs- bzw. Drehwinkelkontrolle verwendet, so ist die Anzahl der Umdrehungen oder die Laufzeit des Motors ein genaues Maß für die geförderte Menge. Als zweiter Sensor ist hier ein Durchflußmesser 14a vorgesehen, der in der Zuleitung von dem Vorratsbehälter 13 zum Einlaß 16 angeordnet ist. Auch hier wird wieder die durchgeflossene Menge mit Sensoren ermittelt, die auf unterschiedlichen physikalischen Meßprinzipien beruhen.

### Menge des umgewälzten Wassers

Auch hier kann die Förderleistung der Umwälzpumpe 5 durch einen Sensor 23 gemessen werden, der beispielsweise die Umdrehungszahl mißt, was bei ansonsten bekannten Parametern, wie Leitungsquerschnitte etc. ein Maß für die umgewälzte Menge ist. Der andere Sensor ist wiederum ein Durchflußmesser 24. Kummulativ kann auch ein Drehsensor 25 verwendet werden, der die Umdrehungen des Dreharmes 8 mißt (nicht Gegenstand der Ereindung). Bei bekannten Leitungsparametern ist nämlich die Drehzahl des Dreharmes 8 wiederum ein Maß für die in der Umwälzleitung 6 umgewälzte Wassermenge. Ergänzend hierzu kann auch ein Drucksensor 26 an die Umwälzleitung 6 angeschlossen sein, aus dessem Meßwert zusammen mit den anderen gemessenen Werten wiederum ein Maß für die umgewälzte Wassermenge ermittelt werden kann (nicht Gegenstand der Erfindung).

### Temperatur, nicht Gegenstand der Ereindung

Für die Messung der Temperatur des aktuellen Waschwassers ist ein Temperaturfühler 27 vorgesehen. Der zweite Meßwert kann durch Erfassung der Heizleistung der Heizeinrichtung 17 ermittelt werden, beispielsweise durch einen Sensor 28, der den von der Heizeinrichtung 17 aufgenommenen Strom mißt. In Kenntnis der im Waschraum befindlichen Wassermenge läßt sich hierüber die Temperatur ermitteln und mit dem Meßwert des Sensors 27 vergleichen.

## Patentansprüche

1. Reinigungs- und Desinfektionsmaschine mit mindestens einem Sensor, der die Menge einer Flüssigkeit mißt,
**dadurch gekennzeichnet,**
**daß** zur Messung der Menge der Flüssigkeit zwei Sensoren (14, 14a) verwendet werden, die je mit unterschiedlichen physikalischen Meßprinzipien arbeiten, wobei
der eine Sensor ein Durchflußmesser (14a, 23) ist und
der andere Sensor die Anzahl der Umdrehungen oder die Laufzeit des Motors einer Dosierpumpe (14) mißt, die ein Maß für die geförderte Menge sind.

## Claims

1. Cleaning and desinfecting machine having at least one sensor measuring the amount of a fluid,
**characterized in that**
for measuring of the amount of the fluid two sensors (14, 14a) are used which work with different physical measuring principles, wherein the one sensor is a flow meter (14a, 23) and the other sensor is measuring the number of revolutions or the runtime of the motor of a dosing pump (14), which are a measurement of the delivered amount.

## Revendications

1. Machine pour le nettoyage et la désinfection comprenant au moins un capteur, qui mesure la quantité d'un liquide,
**caractérisée en ce que**
pour mesurer la quantité du liquide on utilise deux capteurs (14, 14a), qui fonctionnent selon des principes de mesures physiques différents, parmi lesquels
un capteur est un débitmètre (14a, 23) et
l'autre capteur mesure le nombre de rotations ou la durée de fonctionnement du moteur d'une pompe de dosage (14), qui sont une mesure de la quantité souhaitée.
